# EUROPEAN PATENT SPECIFICATION

(11) **EP 2 428 215 B1**
(45) Date of publication and mention of the grant of the patent: **18.10.2017**
(21) Application number: 10772189.6
(22) Date of filing: 07.05.2010
(51) Int. Cl.: A61K 38/00, A61P 9/00, A61P 9/10, A61P 17/02, A61P 43/00

(54) **Recombinant gelatin for use in the treatment of ischemic and heart disease and others**
Rekombinante Gelatine zur Verwendung in der Behandlung von ischämischen und Herzerkrankungen und anderen
Gélatine recombinante pour une utilisation dans le traitement de maladies ischémiques et cardiaques et autres

(30) Priority: 07.05.2009 JP 2009112659
(43) Date of publication of application: 14.03.2012
(73) Proprietor: FUJIFILM Corporation, Tokyo 106-8620 (JP)
(72) Inventor: OGIWARA Kazutaka, Ashigarakami-gun Kanagawa 258-8577 (JP); ISHIKAWA Noriko, Ashigarakami-gun Kanagawa 258-8577 (JP)
(74) Representative: Hoffmann Eitle
(86) International application number: PCT/JP2010/057796
(87) International publication number: WO 2010/128672

(56) References cited:
- EP-A1- 0 702 959
- EP-A1- 2 460 540
- WO-A1-2008/103041
- WO-A1-2008/103046
- WO-A1-2008/133196
- WO-A1-2010/143708
- WO-A2-2004/085473
- JP-A- 2007 297 402
- JP-T- 2003 516 730
- DREESMANN ET AL: "The pro-angiogenic characteristics of a cross-linked gelatin matrix", BIOMATERIALS, ELSEVIER SCIENCE PUBLISHERS BV., BARKING, GB, vol. 28, no. 36, 22 October 2007 (2007-10-22), pages 5536-5543, XP022308852, ISSN: 0142-9612, DOI: 10.1016/J.BIOMATERIALS.2007.08.040
- NEEDLEMAN S B ET AL: "A general method applicable to the search for similarities in the amino acid sequence of two proteins", JOURNAL OF MOLECULAR BIOLOGY, ACADEMIC PRESS, UNITED KINGDOM, vol. 48, no. 3, 28 March 1970 (1970-03-28) , pages 443-453, XP024011703, ISSN: 0022-2836, DOI: 10.1016/0022-2836(70)90057-4 [retrieved on 1970-03-28]

## Description

### Technical Field

The present invention relates to an angiogenesis inducing agent using a recombinant gelatin for use in a method of treatment according to the appended claims.

### Background Art

The term "angiogenesis," mainly refers to a phenomenon in which new blood vessels are formed from existing blood vessels. Examples of normal and physiological angiogenesis include angiogenesis in fetal life and angiogenesis involved in formation of the endometrium/corpus luteum, wound healing or the like. Such an event of angiogenesis is employed in clinical practice for treatment including angiogenesis therapy. It has been elucidated that angiogenesis is important for wound healing, treatment of ischemic diseases and the like and treatment called, in a broad sense, "regenerative medicine" including organ regeneration, cell transplantation, and enhancement of natural healing effects. In order to cause exhibition of original therapeutic effects of angiogenesis or enhancement of therapeutic effects of angiogenesis, therapeutic agents focused on angiogenesis have been developed.

One example of widely available such agents is basic fibroblast growth factor (bFGF). bFGF was identified as a protein capable of strongly stimulating growth of fibroblasts in the bovine pituitary gland. Thereafter, bFGF has been actively studied. As a result, it has been elucidated that it stimulates growth of cells including many types of cells such as vascular endothelial cells, vascular smooth muscle cells, corneal endothelial cells, osteoblasts, and chondrocytes, in addition to fibroblasts. However, the growth factor (i.e., bFGF) is too expensive to be used with an angiogenesis inducing agent In addition, it has been noted that there is a drawback that bFGF might have a risk of causing canceration because of active induction of blood vessels. Therefore, materials that enable induction of blood vessels without growth factors have been awaited.

Meanwhile, biopolymers such as gelatin have been widely used as medical materials. Along with the advancement of gene engineering techniques in recent years, protein synthesis is carried out by introduction of gene into *Escherichia coli* or yeast. With the use of such techniques, different types of recombinant collagen-like proteins have been synthesized (e.g., Patent Documents 1 and 2). As compared with naturally occurring gelatins, the synthetic proteins are superior in non-infectious property, and the synthetic proteins are homogenous and have predetermined sequences and thus they can be precisely designed in terms of strength and degradability. Therefore, the use of such proteins is advantageous. However, as the use of recombinant gelatins suggested in the past, recombinant gelatins have been used as a replacement of naturally occurring gelatin. Needless to say, the use of recombinant gelatins as angiogenesis inducing agent has been unknown.

Dreesmann et al. 2007 discuss the pro-angiogenic characteristics of porcine skin derived cross-linked gelatin matrix.

Patent Document 3 discloses a bFGF formulation which comprises crosslinked gelatin gel. However, gelatin itself is used as a carrier for encapsulating bFGF. It is not taught at all that the gelatin gel itself has a function of causing angiogenesis.

### Prior Art Documents

### Patent Documents

Patent Document 1: US Patent No. 6,992,172
Patent Document 2: WO20081103041
Patent Document 3: WO1994/027630

### Summary of Invention

### Object to be Solved by the Invention

An object of the present invention is to provide a biologically safe and highly bioadhesive angiogenesis inducing agent

### Means for Solving the Object

As a result of intensive studies to achieve the above object, the present inventors found that a recombinant gelatin having an amino acid sequence derived from a partial amino acid sequence of collagen exhibits an action of accumulating at an angiogenesis site and inducing angiogenesis. This has led to the completion of the present invention.

The present invention provides an angiogenesis inducing agent which comprises a recombinant gelatin having an amino acid sequence derived from a partial amino acid sequence of collagen according to the appended claims.

Preferably, the recombinant gelatin comprises repeat of a sequence represented by Gly-X-Y characteristic to collagen and has a molecular weight of 2 KDa to 100 KDa. wherein X and Y each independently represent any amino acid and a plurality of Gly-X-Y sequences may be the same or different.

Preferably, the recombinant gelatin comprises repeat of a sequence represented by Gly-X-Y characteristic to collagen and has a molecular weight of 10 KDa to 90 KDa wherein X and Y each independently represent any amino acid and a plurality of Gly-X-Y sequences may be the same or different.

Preferably, the amino acid sequence of the recombinant gelatin does not comprise any of serine and threonine.

Preferably, the amino acid sequence of the recombinant gelatin does not comprise any of serine, threonine, asparagine, tyrosine, and cysteine.

Preferably, the amino acid sequence of the recombinant gelatin does not comprise an amino acid sequence represented by Asp-Arg-Gly-Asp.

Preferably, the recombinant gelatin is represented by the following formula:

A-[(Gly-X-Y)ₙ]ₘ-B

wherein A represents any amino acid or amino acid sequence, B represents any amino acid or amino acid sequence, there exist n amino acids each independently represented by X, there exist n amino acids each independently represented by Y, n represents an integer from 3 to 100, m represents an integer of 2 to 10, and n Gly-X-Y sequences may be the same or different.

Preferably, the recombinant gelatin is represented by the following formula:

Gly-Ala-Pro-[(Gly-X-Y)₆₃]₃-Gly

wherein there exist 63 amino acids each independently represented by X, there exist 63 amino acids each independently represented by Y, and n Gly-X-Y sequences may be the same or different.

Preferably, the recombinant gelatin has the following (1) or (2):
(1) the amino acid sequence shown in SEQ ID NO: 1; or
(2) an amino acid sequence having 80% or more homology to the amino acid sequence shown in SEQ ID NO: 1, and having an angiogenic action.

Preferably, the recombinant gelatin is crosslinked.

Preferably, crosslinking is carried out using an aldehyde, condensing agent, or enzyme.

Preferably, the angiogenesis inducing agent according to the present invention accumulates at an angiogenesis site so as to induce angiogenesis.

### Advantageous Effects of the Invention

The angiogenesis inducing agent as disclosed herein is characterized in that it comprises a recombinant gelatin, and thus has no risk of causing canceration and is biologically safe and highly bioadhesive.

### Brief Description of Drawings

[Figure 1] Fig. 1 shows the results obtained by determining the amount of hemoglobin in blood for quantitative analysis of angiogenic effects.
[Figure 2] Fig. 2 shows the results of HUVEC adhesiveness test.
[Figure 3] Fig. 3 shows the results of HUVEC adhesiveness test.
[Figure 4] Fig. 4 shows photos showing HUVEC cells on plates coated with different proteins.
[Figure 5] Fig. 5 shows a comparison of 1-cell area for HUVEC cells on plates coated with different proteins.
[Figure 6] Fig. 6 shows HUVEC cell adhesion inhibitory effects of an anti-αV antibody.

### Embodiments for Carrying out the Invention

Embodiments for carrying out the present invention are described in detail below.

As a recombinant gelatin used in the present invention, a recombinant gelatin having an amino acid sequence derived from a partial amino acid sequence of collagen can be used. Examples of a recombinant gelatin that are disclosed herein include, but are not limited to, recombinant gelatins described in EP101476A2, US6992172, WO2004-8547 and WO2008/103041. A recombinant gelatin preferably used as the recombinant gelatin of the present invention is described below.

The recombinant gelatin used in the present invention has original properties of naturally occurring gelatin and thus it is highly biocompatible. In addition, the recombinant gelatin is not directly obtained from natural sources and thus has no risk of causing BSE or the like. In this regard, it has an excellent property of being non-infectious. In addition, the recombinant gelatin used in the present invention is more homogenous than naturally occurring gelatin. Further, the recombinant gelatin has a predetermined sequence. Thus, it is possible to precisely design the recombinant gelatin in terms of strength and degradability with few errors by crosslinking or the like described below.

The molecular weight of the recombinant gelatin used in the present invention is preferably 2 KDa to 100 KDa, more preferably 2.5 KDa to 95 KDa, further preferably 5 KDa to 90 KDa, and most preferably 10 KDa to 90 KDa.

The recombinant gelatin used in the present invention contains repeat of a sequence represented by Gly-X-Y characteristic to collagen. Here, a plurality of sequences each represented by Gly-X-Y may be the same or different. Gly in Gly-X-Y represents glycine. X and Y in Gly-X-Y represent any amino acid (and preferably any amino acids other than glycine). When gelatin/collagen is compared with other proteins in terms of the amino acid composition or sequence, the GXY sequence characteristic to collagen is a highly specific partial structure. Glycine accounts for approximately one-third of the partial structure as a whole. Glycine is repeatedly found in the amino acid sequence at a rate of 1 out of every 3 amino acids. Glycine is the simplest amino acid. There are few restrictions to arrangement of the molecular chain of glycine and thus glycine highly contributes to regeneration of the helix structure upon gelatinization. Preferably, an amino acid represented by X or Y is rich in imino acid (proline or oxyproline) and the imino acid accounts for 10% to 45% of the amino acid sequence as a whole. Amino acids forming the GXY repeat structure account for preferably 80% or more, more preferably 95% or more, and most preferably 99% or more of the amino acid sequence as a whole.

A generally available gelatin contains charged polar amino acids and uncharged polar amino acids at a ratio of 1:1. Here, the term "polar amino acid" specifically refers to cysteine, aspartic acid, glutamic acid, histidine, lysine, asparagine, glutamine, serine, threonine, tyrosine, or arginine. In particular, the term "uncharged polar amino acid" refers to cysteine, asparagine, glutamine, serine, threonine, or tyrosine. The percentage of polar amino acids relative to all amino acids constituting the recombinant gelatin used in the present invention is 10% to 40% and preferably 20% to 30%. In addition, the percentage of uncharged polar amino acids relative to the polar amino acids is preferably 5% to less than 20% and more preferably less than 10%. Further, the amino acid sequence does not contain one amino acid and preferably two amino acids or more selected from among serine, threonine, asparagine, tyrosine, and cysteine.

In general, it is known that a polypeptide contains a minimal amino acid sequence that functions as a cell adhesion signal (e.g., "Pathophysiology" (Byotai Seiri) Vol. 9, No. 7 (1990), p. 527, Nagai Shoten Co., Ltd.). It is preferable for a single molecule of the recombinant gelatin used in the present invention to have at least two cell adhesion signal sequences. Specifically, amino acids are shown by one-letter notation in a cell adhesion signal sequence. In view of an increase in types of adhering cells, examples of such sequence are: preferably an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence, more preferably an RGD sequence, a YIGSR sequence, a PDSGR sequence, an LGTIPG sequence, an IKVAV sequence, and an HAV sequence.

An example of an RGD sequence is ERGD.

In terms of arrangement of ERGD sequences in the recombinant gelatin used in the present invention, the number of amino acids present between two ERGD sequences is preferably 0 to 100 and more preferably 25 to 60. Preferably, the number of amino acids is not unifonnly determined.

In view of cell adhesion/growth, the number of the minimal amino acid sequences in a single protein molecule is preferably 3 to 50, more preferably 4 to 30, particularly preferably 5 to 20, and most preferably 12.

The percentage of ERGD motifs in the recombinant gelatin used in the present invention related to the total number of amino acids is preferably at least 0.4%. If the recombinant gelatin comprises 350 amino acids or more, each stretch of 350 amino acids contains preferably at least one ERGD motif. The percentage of ERGD motifs related to the total number of amino acids is more preferably at least 0.6%, further preferably at least 0.8%, still further preferably at least 1.0%, even further preferably at least 1.2%, and most preferably at least 1.5%. The number of ERGD motifs in the recombinant gelatin is preferably at least 4, more preferably 6, further preferably 8, and even further preferably 12 to 16 per 250 amino acids. A percentage of ERGD motifs of 0.4% corresponds to at least one ERGD sequence per 250 amino acids. The number of ERGD motifs is represented by an integer. Therefore, in order to achieve a percentage of ERGD motifs of 0.4%, it is necessary for a gelatin comprising 251 amino acids to contain at least two ERGD sequences. Preferably, the recombinant gelatin used in the present invention contains at least 2 ERGD sequences per 250 amino acids, more preferably at least 3 ERGD sequences per 250 amino acids, and further preferably at least 4 ERGD sequences per 250 amino acids. In another embodiment, the recombinant gelatin of the present invention comprises at least 4, preferably 6, more preferably 8, and further preferably 12 to 16 ERGD motifs.

In addition, the recombinant gelatin may be partially hydrolyzed.

Preferably, the recombinant gelatin used in the present invention has a structure comprising repeat of A-[(Gly-X-Y)ₙ]ₘ-B. Here, "m" is an integer of preferably 2 to 10 and more preferably 3 to 5. In addition, "n" is an integer of preferably 3 to 100, more preferably 15 to 70, and most preferably 50 to 65.

Preferably, a plurality of naturally occurring collagen sequence units are bound to form a repeat unit. The term "naturally occurring collagen" used herein may refer to any naturally occurring collagen. However, preferable examples thereof include type-I, type-II, type-III, type-IV, and type-V collagens. More preferably, type-I, type-II, and type-III collagens are used. In another embodiment, the origin of the collagen is preferably a human, bovine, pig, mouse, or rat and it is more preferably a human.

The isoelectric point of the recombinant gelatin used in the present invention is preferably 5 to 10, more preferably 6 to 10, and further preferably 7 to 9.5.

Preferably, the recombinant gelatin is not deaminated.

Preferably, the recombinant gelatin is not procollagen or does not comprise procollagen.

Preferably, the recombinant gelatin does not comprise telopeptide.

Preferably, the recombinant gelatin is a substantially pure collagen material prepared from a nucleic acid encoding a naturally occurring collagen.

Particularly preferably, the recombinant gelatin used in the present invention is a recombinant gelatin having the following (1) or (2):
(1) the amino acid sequence shown in SEQ ID NO: 1; or
(2) an amino acid sequence having 80% or more, more preferably 90% or more, and most preferably 95% or more homology to the amino acid sequence shown in SEQ ID NO: 1, and having an angiogenic action.

The recombinant gelatin used in the present invention can be produced by a gene recombination technique known to persons skilled in the art. For instance, it can be produced according to the method described in EP1014176A2, US6992172, WO2004/85473, or WO2008/103041. Specifically, a transformant is produced by obtaining a gene encoding the amino acid sequence of certain recombinant gelatin, incorporating the gene into an expression vector to prepare a recombinant expression vector, and introducing the vector into an appropriate host. The obtained transformant is cultured in an appropriate medium to produce a recombinant gelatin. Therefore, the recombinant gelatin used in the present invention can be prepared by collecting the produced recombinant gelatin from the culture product.

If the obtained recombinant gelatin alone has insufficient properties, it may be mixed with other material, or a complex of recombinant gelatin and other material may be prepared. For example, it can be mixed with a different type of recombinant gelatin or a different biopolymer or synthetic polymer. Examples of a biopolymer include a polysaccharide, a polypeptide, a protein, a nucleic acid, and an antibody. Preferably, a polysaccharide, a polypeptide, or a protein is used. Examples of a polysaccharide, a polypeptide and a protein include collagen, gelatin, albumin, fibroin, and casein. Further, the above biopolymers may be partially chemically modified according to need. For instance, hyaluronic acid ethyl ester can be used. Examples of a polysaccharide include glycosaminoglycan represented by hyaluronic acid or heparin, chitin, and chitosan. Further, examples of a polyarnino acid include poly-γ-glutamic acid.

The recombinant gelatin used in the present invention can be chemically modified depending on the application thereof. Chemical modification may be performed via introduction of a low molecular compound or a different polymer (e.g., a biopolymer (sugar or protein), a synthetic polymer, or polyamide) into a carboxyl group or an amino group of a side chain of the recombinant gelatin, or crosslinking between recombinant gelatin chains. For example, a carbodiimide-based condensing agent is used for introduction of a low molecular compound into the recombinant gelatin.

The crosslinking agent used in the present invention is not particularly limited as long as the present invention can be carried out. It may be a chemical crosslinking agent or an enzyme. Examples of a chemical crosslinking agent include formaldehyde, glutaraldehyde, carbodiimide, and cyanamide. Preferably, formaldehyde or glutaraldehyde is used. Further, crosslinking of a recombinant gelatin can be conducted by light irradiation to a gelatin into which a photoreactive group has been introduced, light irradiation under the presence of a photosensitizer, or the like. Examples of a photoreactive group include a cinnamyl group, a coumarin group, a dithiocarbamyl group, xanthene dye, and camphorquinone.

In a case in which enzymatic crosslinking is carried out, an enzyme used is not particularly limited as long as it has an action of causing crosslinking between recombinant gelatin chains. However, crosslinking can be carried out using preferably transglutaminase or laccase and most preferably transglutaminase. Examples of proteins that are enzymatically crosslinked by transglutaminase include, but are not particularly limited to, proteins having lysine residues and glutamine residues. A mammalian-derived or microorganism-derived transglutaminase may be used. Specific examples thereof include: the Activa series (produced by Ajinomoto Co., Inc.); commercially available mammalian-derived transglutaminases serving as reagents such as guinea pig liver-derived transglutaminase, goat-derived transglutaminase, and rabbit-derived transglutaminase (produced by Oriental Yeast Co., Ltd., Upstate USA Inc., Biodesign International, etc.); and a human-derived blood coagulation factor (Factor XIIIA, Haematologic Technologies, Inc.).

Crosslinking of the recombinant gelatin comprises the following two steps: a step of mixing a recombinant gelatin solution and a crosslinking agent; and a step of causing a reaction in the obtained homogenous solution.

According to the present invention, the mixing temperature for treating the recombinant gelatin with a crosslinking agent is not particularly limited as long as the solution can be homogenously agitated. However, it is preferably 0°C to 40°C, more preferably 0°C to 30°C, further preferably 3°C to 25°C, still further preferably 3°C to 15°C, even further preferably 3°C to 10°C, and particularly preferably 3°C to 7°C.

After agitation of the recombinant gelatin and the crosslinking agent, the temperature can be increased. The reaction temperature is not particularly limited as long as crosslinking can proceed. However, in view of denaturation or degradation of the recombinant gelatin, it is substantially 0°C to 60°C, preferably 0°C to 40°C, more preferably 3°C to 25°C, further preferably 3°C to 15°C, still further preferably 3 °C to 10°C, and particularly preferably 3°C to 7°C.

If necessary, it is possible to encapsulate a drug with the recombinant gelatin used in the present invention. The drug is a physiologically active ingredient. Specific examples thereof include percutaneous absorbents, topical therapeutic agents, oral therapeutic agents, cosmetic ingredients, and supplement ingredients. Specific examples of a drug include anti-inflammatory agents, antibacterial agents, antibiotics, immunosuppressive agents, antioxidants, anticancer agents, vitamins, nucleic acids, and antibodies. Particularly preferred examples thereof are anti-inflammatory agents. Both steroidal and nonsteroidal anti-inflammatory agents may be used. Examples of anti-inflammatory agents include aspirin, acetaminophen, phenacetin, indomethacin, diclofenac sodium, piroxicam, fenoprofen calcium, ibuprofen, chlorpheniramine maleate, diflunisal, dexamethasone sodium phosphate, paclitaxel, docetaxel, 5-fluorouracil, Topotecin, cisplatin, rapamycin, tacrolimus, and cyclosporin. Vitamins that can be used may be water-soluble vitamins or fat-soluble vitamins. Examples of such vitamins include vitamin A, the vitamin B group, vitamin C, the vitamin D group, vitamin E, and vitamin K. Specific examples of drugs are described above. However, as long as the recombinant gelatin used in the present invention is applied, drugs that can be used in the present invention are not limited to the above drugs.

According to the present disclosure angiogenesis can be induced by administering the above recombinant gelatin having an amino acid sequence derived from a partial amino acid sequence of collagen into a subject in need of angiogenesis induction (e.g., a mammal such as a human).

The dose, the usage, and the dosage form of the angiogenesis inducing agent of the present invention can be appropriately determined depending of the purpose of use. For example, the angiogenesis inducing agent of the present invention can be directly administered *in vivo* to a desired site. Alternatively, it may be suspended in a liquid excipient such as an aqueous solvent (e.g., distilled water for injection, physiological saline for injection, or buffer (e.g., phosphate or citrate buffer) (pH 5 to 8)) so as to be administered via injection, external application, or the like. In addition, it may be mixed with an adequate excipient in the form of ointment, gel, cream, or the like so as to be externally applied. That is, the administration route of the angiogenesis inducing agent of the present invention may be the oral route or the parenteral route (e.g., intravenous administration, intramuscular administration, subcutaneous administration, or intradermal administration). In addition, for treatment of coronary artery diseases, peripheral arteriosclerosis obliterans, anangioplasia, etc., the angiogenesis inducing agent of the present invention can be directly injected into cardiac muscle via a ventricular cavity using a catheter or it can be locally released or applied to a stenosis or obstruction lesion in a coronary artery using a catheter. Examples of the dosage form include: oral administration agents such as tablets, powders, capsules, granules, extracts, and syrups; and parenteral administration agents such as parenteral injections (e.g., intravenous injections, muscular injections, subcutaneous injections, and intradermal injections). For example, when the angiogenesis inducing agent of the present invention is locally administered, the dosage form thereof is not particularly limited. However, it can be formed into sponge, film, unwoven fabric, fibers (tubes), particles, mesh, or the like.

A formulation of the angiogenesis inducing agent of the present disclosure can be prepared by a method known to persons skilled in the art. For example, if liquid is used as a carrier for a formulation, the angiogenesis inducing agent of the present invention can be dissolved or dispersed in the liquid. Alternatively, if a powder is used as a carrier for a formulation, the angiogenesis inducing agent of the present invention can be mixed with or adhere to the powder. Further, if necessary, a pharmaceutically acceptable additive (e.g., a preservative, a stabilizer, an antioxidant, an excipient, a binder, a disintegrator, a wetting agent, a lubricant, a coloring agent, an aromatic agent, a corrigent, a coating, a suspending agent, an emulsifier, a dissolution adjuvant, a buffer, a tonicity agent, plasticizer, a surfactant, or a soothing agent) can be mixed therewith.

The administration dose of the recombinant gelatin is not particularly limited. However, for example, it can be 1 to 100 mg and preferably 1 to 50 mg per 1-cm² surface area of a subject organism.

Examples of diseases that can be treated according to the present invention include: ischemic diseases; diabetic gangrene; ulcer; hearing loss; heart diseases; arteriosclerosis; acute coronary syndromes; acute myocardial infarction; unstable angina pectoris; and cardiac sudden death.

The present invention is hereafter described in greater detail with reference to the following examples, although the present invention is not limited thereto.

### Examples

As a recombinant gelatin, CBE3 (WO2008-103041) described below was prepared.
CBE3
Molecular weight: 51.6 kD
Structure: GAP[(GXY)63]3G
Number of amino acids: 571
Number of RGD sequences: 12
Imino acid content: 33%
(Substantially 100% of amino acids form the GXY repeat structure. The amino acid sequence of CBE3 does not contain any of serine, threonine, asparagine, tyrosine, and cysteine. CBE3 has the ERGD sequence.)
Isoelectric point: 9.34

Amino acid sequence (SEQ ID NO: 1 in the Sequence Listing) (This amino acid sequence corresponds to the amino acid sequence shown in SEQ ID NO: 3 in WO2008/103041. Note that "X" at the end was modified to "P.")

In the Examples described below, CBE3 above used as a recombinant gelatin is described as "R-Gel" unless specified otherwise.

### (1) Angiogenesis test using mice

An aqueous solution containing glutaraldehyde (Wako Pure Chemical Industries, Ltd.) (1%) (0.27 ml) was added to an R-Gel aqueous solution (5.56%) (2.43 ml). The resulting solution was introduced into a silicone mold (3 cm x 3 cm x 4 mm) and left at room temperature for 1 hour. Thereafiler, a crosslinking reaction was induced by maintaining the temperature at 4°C for 24 hours. After the reaction the obtained gel was removed from the mold. The gel was immersed in a 0.1M glycine aqueous solution (30 ml) to terminate the crosslinking reaction. The obtained crosslinked R-Gel was washed three times with distilled water at room temperature for 1 hour. The obtained gel was rapidly cooled at -50°C, followed by lyophilization at -10°C for 48 hours. The water content in gel was determined based on changes in the amount of crosslinked R-Gel before and after PBS swelling treatment at 4°C for 24 hours. It was found to be 95.4%. A portion of the obtained gel (5 mg) was excised.

The thus prepared R-Gel formulation (5 mg) impregnated with a PBS solution (50 µl) was subcutaneously embedded in dorsal regions of mince. For a control group, a PBS solution (50 µl) alone was subcutaneously administered to mice. For another control group, PI-5 gel (MedGEL Corporation) (5 mg) impregnated with a PBS solution (50 µl) was subcutaneously embedded in the same manner. Three days after administration, the skin of each mouse was peeled off. Each lesion into which the formulation had been embedded or a PBS solution had been administered was observed. For the PBS solution administration group, the state of tissue surrounding the administration site was similar to that of the corresponding tissue of the untreated group. Thus, no changes were visually confirmed. For the PI-5 gel administration group, substantially no angiogenesis image was observed for the gel-embedded lesions as in the case of the untreated group. However, for the R-Gel-formulation-embedded group, it was visually confirmed that the tissue surrounding the formulation-embedded site became red. Thus, angiogenic effects that were thought to be derived from high affinity between RGD-rich R-Gel and vascular endothelial cells were obviously confirmed.

The above results show that efficient adhesion of vascular endothelial cells was unable to take place in the PI-5 gel administration group. Meanwhile, the use of the R-Gel formulation of the present invention promoted adhesion of vascular endothelial cells without growth factors such as bFGF, allowing exhibition of angiogenic effects.

For quantitative determination of angiogenic effects, the hemoglobin amount in blood was determined. Hemoglobin B-test Wako kits (Wako Pure Chemical Industries, Ltd.) were used for determination.

The prepared R-Gel formulation (5 mg) impregnated with a PBS solution (50 µl) was subcutaneously embedded in dorsal regions of mice. For a control group, a PBS solution (50 µl) alone was subcutaneously administered to mice. For another control group, PI-5 gel (MedGEL Corporation) (5 mg) impregnated with a PBS solution (50 µl) was subcutaneously embedded in the same manner. Two weeks after administration, the skin of each mouse was peeled off. A piece (1.5 cm x 1.5 cm) of tissue surrounding a lesion into which the formulation had been embedded or a PBS solution had been administered was excised from each skin sample and placed in a tube. Each piece of the tissue was finely cut in the tube. An extraction liquid (10 mM Tris, 1 mM EDTA, pH 7.8) (300 µl) was added thereto, followed by overnight agitation at 4°C using a rotator. Then, centrifugation was performed to separate the supernatant The hemoglobin amount was determined using the supernatant in accordance with the protocols of the kit.

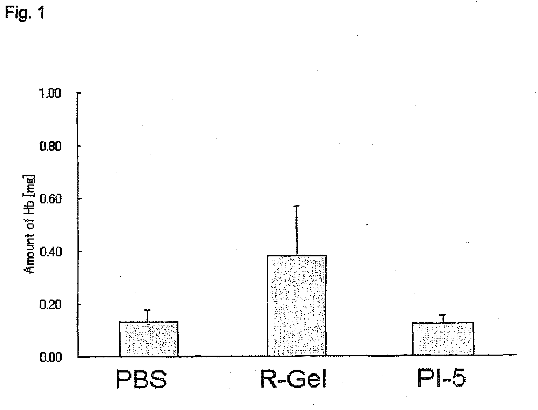

The results are shown in Fig. 1. The results show that the hemoglobin amount of the R-Gel formulation administration group obviously increased to a greater extent than that of the PBS solution administration group and that of the PI-5 gel administration group. Therefore, R-Gel was confirmed to have angiogenic effects.

### (2) Cell adhesiveness test (for interaction between R-Gel and αVβ3 integrin)

In order to elucidate the mechanism of R-Gel to cause accumulation of newly formed vessels, tests were conducted to examine cell adhesiveness of R-Gel to vascular endothelial cells and experiments were conducted to examine interaction between R-Gel and αVβ3 integrin.

HUVECs (normal human umbilical vein endothelial cells; Takara Bio Inc.) were used as vascular endothelial cells. It is known that many αVβ3 integrin molecules are permanently expressed on the surfaces of HUVECs. By testing cell adhesiveness of R-Gel to HUVECs, binding of R-Gel to vascular endothelial cells activated by newly formed vessels can be elucidated. Also, binding of R-Gel to αVβ3 integrin that has been reported to be highly expressed at newly formed vessel sites can be elucidated.

For culture of HUVECs, endothelial cell basic medium-2 (containing no serum) (EBM™-2) and endothelial cell medium kit-2 (2% FBS) (EGM™-2 BulletKit™) were used (Takara Bio Inc.). Upon subculture and removal of cells, an EDTA-containing 0.25% trypsin solution was used. HUVECs that had been allowed to proliferate to a sufficient amount in a T-75 flask were removed from the bottom surface of the flask, followed by centrifugation for removal of the supernatant. Then, the resultant was washed with the above endothelial cell basic medium-2 containing the endothelial cell medium kit 2. Centrifugation was performed again to remove the supernatant. A solution obtained by adding 0.1% BSA to endothelial cell basic medium-2 containing no endothelial cell medium kit 2 was added thereto for suspension. The number of living cells was counted using cell counter. The final cell concentration was adjusted to 500,000 cells/mL.

Meanwhile, plates coated with various proteins (R-Gel, fibronectin, collagen manufactured by Fibrogen (heteinafter referred to as "Fibrogen"), pig skin gelatin (hereinafter referred to as "PSK"), and beef-bone-derived gelatin (hereinafter referred to as "G1917P")) were prepared for cell adhesiveness tests. R-Gel was dissolved in PBS (phosphate buffer) at a concentration of 1 mg/mL to prepare an R-Gel solution. Fibronectin was dissolved in PBS (phosphate buffer) at a concentration of 1 mg/mL to prepare a fibronectin solution. Fibrogen was dissolved in PBS (phosphate buffer) at a concentration of 1 mg/mL to prepare a fibrogen solution. PSK was dissolved in PBS (phosphate buffer) at a concentration of 1 mg/mL to prepare a PSK solution. G1917P was dissolved in PBS (phosphate buffer) at a concentration of 1 mg/mL to prepare a G1917P solution. The above solutions were diluted with PBS according to need and used as the solutions to be added to plates.

Non-treated 96-well plates (IWAKI) were used as plates. Solutions obtained by diluting the above solutions with PBS were added to non-treated 96-well plates (50 µL/well) so as to result in protein concentrations of 0.02, 0.1, 0.2, and 2.0 µg/well. Then, incubation was carried out at 37°C for 2 hours. After removal of each solution, PBS (100 µL) was added to every well, followed by washing for removal of PBS (a washing step). The washing step was repeated 3 times. Accordingly, plates coated with different coating proteins at different coating concentrations were obtained.

The HUVEC suspension (500,000 cells/mL) prepared above was inoculated on the coating plates (100 µL each), followed by incubation at 37°C for 1 hour. Then, the medium was removed by suction, followed by washing with PBS (100 µL). PBS was removed by suction (PBS washing). PBS washing was repeated 3 times in the above manner. Thus, the plates from which PBS had been removed were obtained.

DNA assay was performed for quantitative determination of the number of cells on the obtained plates. 100 µl of SDS solution (i.e., a solution obtained by dissolving 20 mg of SDS in 100 mL of 1 x SSC solution (obtained by dissolving 17.999 g of NaCl and 8.823 g of Na₃-citrate in 2L of ultrapure water)) was added to wells of the obtained plates. Each plate was allowed to stand still at 37°C for 1 hour. The total amount of each obtained solution was transferred to a 96-well black plate (Non-treated) and 100 µL of a Hoechst solution (obtained by mixing 20 µl of Hoechst 33258 with 20 mL of a 1 x SSC solution) was added to every well, followed by determination of the fluorescence intensity using a plate reader. The fluorescence intensity was measured using a Gemini EM plate reader (Molecular Devices Corporation) at an excitation wavelength of 355 nm and a determination wavelength of 460 nm. A calibration curve was created using a suspension containing HUVECs at the adjusted number of cells.

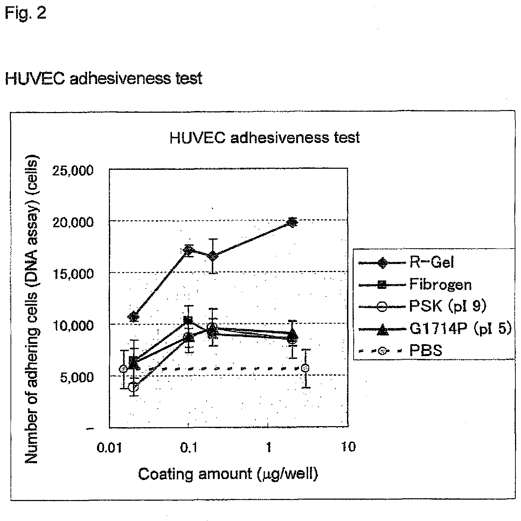

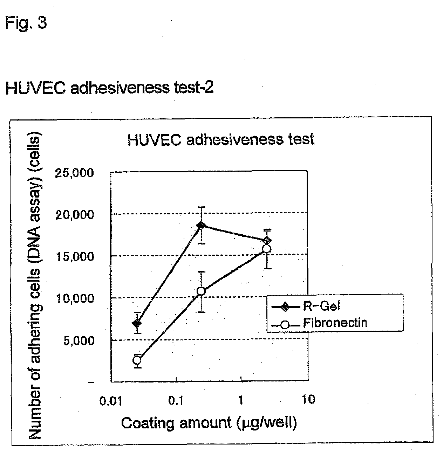

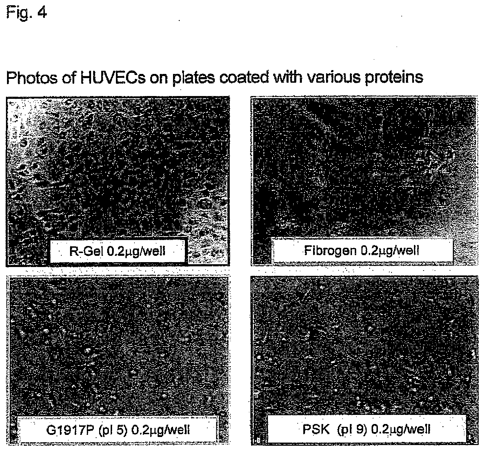

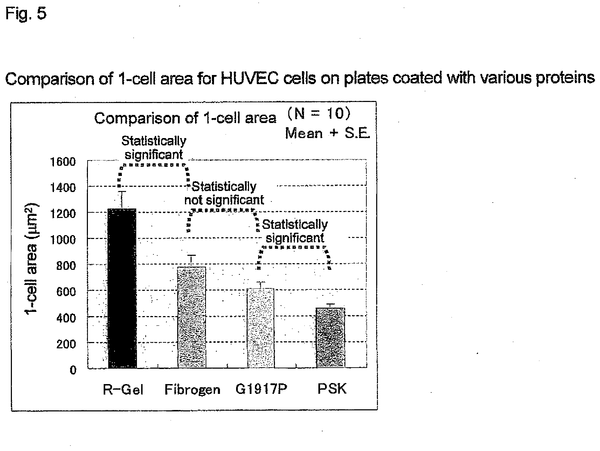

Figs. 2 and 3 show the obtained results of cell adhesiveness tests (by DNA assay). The results showed that R-Gel is superior to fibronectin, Fibrogen, PSK, and G1917P in terms of adhesion to HUVECs. In addition, Fig 4 shows a photo of cell adhesion on the R-Gel-coated plate, a photo of cell adhesion on the Fibrogen-coated plate, a photo of cell adhesion on the PSK-coated plate, and a photo of cell adhesion on the G1917P-coated plate. It can be visually confirmed that the largest number of cells adhered to the R-Gel-coated plate. At the same time, the area of a single cell separately adhering to a plate was determined based on the photos using the ImageJ software. Fig. 5 shows the results. The results showed that R-Gel is significantly superior to Fibrogen. PSK, and G1917P in terms of 1-cell area. Therefore, it was found that binding between R-Gel and HUVECs takes place more strongly than binding in the other cases.

### (3) Experimentation of inhibition of αVβ3-integrin-mediated HUVECs adhesion

In order to identify binding between R-Gel and HUVECs as αVβ3-integrin-mediated binding, experimentation was conducted to determine whether or not R-Gel-to-cell adhesion would be inhibited by blocking αVβ3 integrin with an anti-αV antibody in the R-Gel-to-cell adhesiveness tests conducted in (2) above.

Specifically, cell adhesion experiments were conducted as described in (2) above. Experiments were conducted using the R-Gel-coated plate and the fibronectin-coated plate at a coating concentration of 0.2 µg/well. The prepared HUVECs were incubated with anti-human αV monoclonal antibodies (MAB1980: CHEMICON) at a sufficient concentration at 37°C for 30 minutes, and they are described as "antibody-treated HUVECs". On the other hand, the prepared HUVECs were incubated with the addition of the same amount of PBS at 37°C for 30 minutes, and they are described as "untreated HUVECs". Cells were inoculated on plates with addition of a solution (100 µL/well) prepared in a manner such that it contained antibody-treated HUVECs or untreated HUVECs at a concentration of 1,000,000 cells/mL. The time for cell adhesion was determined to be 1 hour at 37°C as in the case of (2) above. Quantitative determination of the number of cells was carried out by DNA assay as in the case of (2) above.

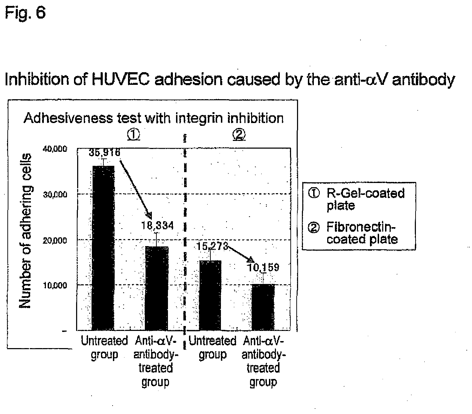

The obtained results are shown in Fig. 6. The results showed that adhesion of R-Gel and fibronectin to HUVECs is significantly inhibited by an anti-human αV antibody. It is known that fibronectin binds to HUVECs via αVβ3 integrin. In this Example, it was shown that R-Gel also binds to HUVECs via αVβ3 integrin as in the case of fibronectin.

This indicates that R-Gel binds to αVβ3 integrin. The results obtained in (2) and (3) above indicate that R-Gel binds to αVβ3 integrin with good efficiency and the binding is stronger than binding in the cases of other collagens/gelatins, and that R-Gel binds to vascular endothelial cells with good efficiency and the binding is stronger than binding in the cases of other collagens/gelatins. That is, the results showed R-Gel has a high ability to bind to newly formed vessels in a highly specific manner at cellular/molecular levels.

As a result of the above experiments, it was shown that a recombinant gelatin binds to vascular endothelial cells with good efficiency and induces angiogenesis.

## Claims

1. Recombinant gelatin having an amino acid sequence derived from a partial amino acid sequence of collagen for use in a method of treatment of ischemic diseases, diabetic gangrene, ulcer, hearing loss, heart diseases, arteriosclerosis, acute coronary syndromes, acute myocardial infarction, unstable angina pectoris, and cardiac sudden death by inducing angiogenesis, wherein the recombinant gelatin comprises repeats of a sequence represented by Gly-X-Y characteristic to collagen and a single molecule of the recombinant gelatin has at least two cell adhesion signal sequences wherein X and Y each independently represent any amino acid and a plurality of Gly-X-Y sequences may be the same or different, and wherein the cell adhesion signal sequence is an amino acid sequence represented by Glu-Arg-Gly-Asp.

2. The recombinant gelatin for use according to claim 1, wherein the recombinant gelatin comprises repeats of a sequence represented by Gly-X-Y characteristic to collagen and has a molecular weight of 2 KDa to 100 KDa wherein X and Y each independently represent any amino acid and a plurality of Gly-X-Y sequences may be the same or different.

3. The recombinant gelatin for use according to claim 1 or 2, wherein the recombinant gelatin comprises repeats of a sequence represented by Gly-X-Y characteristic to collagen and has a molecular weight of 10 KDa to 90 KDa wherein X and Y each independently represent any amino acid and a plurality of Gly-X-Y sequences may be the same or different.

4. The recombinant gelatin for use according to any one of claims 1 to 3, wherein the amino acid sequence of the recombinant gelatin does not comprise any of serine and threonine.

5. The recombinant gelatin for use according to any one of claims 1 to 4, wherein the amino acid sequence of the recombinant gelatin does not comprise any of serine, threonine, asparagine, tyrosine, and cysteine.

6. The recombinant gelatin for use according to any one of claims 1 to 5, wherein the amino acid sequence of the recombinant gelatin does not comprise an amino acid sequence represented by Asp-Arg-Gly-Asp.

7. The recombinant gelatin for use according to any one of claims 1 to 6, wherein the recombinant gelatin is represented by the following formula:
A-[(Gly-X-Y)ₙ]ₘ-B
wherein A represents any amino acid or amino acid sequence, B represents any amino acid or amino acid sequence, there exist n amino acids each independently represented by X, there exist n amino acids each independently represented by Y, n represents an integer from 3 to 100, m represents an integer of 2 to 10, and n Gly-X-Y sequences may be the same or different.

8. The recombinant gelatin for use according to any one of claims 1 to 7, wherein the recombinant gelatin is represented by the following formula:
Gly-Ala-Pro-[(Gly-X-Y)₆₃]₃-Gly
wherein there exist 63 amino acids each independently represented by X, there exist 63 amino acids each independently represented by Y, and 63 Gly-X-Y sequences may be the same or different.

9. The recombinant gelatin for use according to any one of claims 1 to 8, wherein the recombinant gelatin has the amino acid sequence shown in SEQ ID NO: 1.

10. The recombinant gelatin for use according to any one of claims 1 to 9, wherein the recombinant gelatin is crosslinked.

11. The recombinant gelatin for use according to claim 10, wherein crosslinking is carried out using an aldehyde, condensing agent, or enzyme.

12. The recombinant gelatin for use according to any one of claims 1 to 11, which accumulates at an angiogenesis site so as to induce angiogenesis.

13. Use of a recombinant gelatin having an amino acid sequence derived from a partial amino acid sequence of collagen for production of an angiogenesis inducing medicament for the treatment of ischemic diseases, diabetic gangrene, ulcer, hearing loss, heart diseases, arteriosclerosis, acute coronary syndromes, acute myocardial infarction, unstable angina pectoris, and cardiac sudden death, wherein the recombinant gelatin comprises repeats of a sequence represented by Gly-X-Y characteristic to collagen and a single molecule of the recombinant gelatin has at least two cell adhesion signal sequences wherein X and Y each independently represent any amino acid and a plurality of Gly-X-Y sequences may be the same or different, and wherein the cell adhesion signal sequence is an amino acid sequence represented by Glu-Arg-Gly-Asp.

## Patentansprüche

1. Rekombinante Gelatine mit einer Aminosäuresequenz, abgeleitet von einer partiellen Aminosäuresequenz von Kollagen, zur Verwendung in einem Verfahren zur Behandlung von ischämischen Erkrankungen, diabetischem Gangrän, Geschwür, Hörverlust, Herzerkrankungen, Arteriosklerose, akuten Koronarsyndromen, akutem Myokardinfarkt, instabiler Angina pectoris und plötzlichem Herztod durch Induzieren von Angiogenese, wobei die rekombinante Gelatine Wiederholungen einer durch Gly-X-Y repräsentierten, für Kollagen charakteristischen Sequenz umfasst und ein einzelnes Molekül der rekombinanten Gelatine wenigstens zwei Zelladhäsionssignalsequenzen aufweist, wobei X und Y jeweils unabhängig voneinander irgendeine Aminosäure repräsentieren und eine Mehrzahl von Gly-X-Y-Sequenzen die gleichen oder unterschiedlich sein können, und wobei die Zelladhäsionssignalsequenz eine Aminosäuresequenz, repräsentiert durch Glu-Arg-Gly-Asp, ist.

2. Rekombinante Gelatine zur Verwendung gemäß Anspruch 1, wobei die rekombinante Gelatine Wiederholungen einer durch Gly-X-Y repräsentierten, für Kollagen charakteristischen Sequenz umfasst und ein Molekulargewicht von 2 kDa bis 100 kDa hat, wobei X und Y jeweils unabhängig voneinander irgendeine Aminosäure repräsentieren und eine Mehrzahl von Gly-X-Y-Sequenzen die gleichen oder unterschiedlich sein können.

3. Rekombinante Gelatine zur Verwendung gemäß Anspruch 1 oder 2, wobei die rekombinante Gelatine Wiederholungen einer durch Gly-X-Y repräsentierten, für Kollagen charakteristischen Sequenz umfasst und ein Molekulargewicht von 10 kDa bis 90 kDa hat, wobei X und Y jeweils unabhängig voneinander irgendeine Aminosäure repräsentieren und eine Mehrzahl von Gly-X-Y-Sequenzen die gleichen oder unterschiedlich sein können.

4. Rekombinante Gelatine zur Verwendung gemäß irgendeinem der Ansprüche 1 bis 3, wobei die Aminosäuresequenz der rekombinanten Gelatine keines von Serin und Threonin umfasst.

5. Rekombinante Gelatine zur Verwendung gemäß irgendeinem der Ansprüche 1 bis 4, wobei die Aminosäuresequenz der rekombinanten Gelatine keines von Serin, Threonin, Asparagin, Tyrosin und Cystein umfasst.

6. Rekombinante Gelatine zur Verwendung gemäß irgendeinem der Ansprüche 1 bis 5, wobei die Aminosäuresequenz der rekombinanten Gelatine keine durch Asp-Arg-Gly-Asp repräsentierte Aminosäuresequenz umfasst.

7. Rekombinante Gelatine zur Verwendung gemäß irgendeinem der Ansprüche 1 bis 6, wobei die rekombinante Gelatine durch die folgende Formel repräsentiert wird:
A-[(Gly-X-Y)ₙ]ₘ-B,
wobei A irgendeine Aminosäure oder Aminosäuresequenz repräsentiert, B irgendeine Aminosäure oder Aminosäuresequenz repräsentiert, es n Aminosäuren gibt, die jeweils unabhängig voneinander durch X repräsentiert werden, es n Aminosäuren gibt, die jeweils unabhängig voneinander durch Y repräsentiert werden, n eine ganze Zahl von 3 bis 100 repräsentiert, m eine ganze Zahl von 2 bis 10 repräsentiert und n Gly-X-Y-Sequenzen die gleichen oder unterschiedlich sein können.

8. Rekombinante Gelatine zur Verwendung gemäß irgendeinem der Ansprüche 1 bis 7, wobei die rekombinante Gelatine durch die folgende Formel repräsentiert wird:
Gly-Ala-Pro[(Gly-X-Y)₆₃]₃-Gly,
wobei es 63 Aminosäuren gibt, die jeweils unabhängig voneinander durch X repräsentiert werden, es 63 Aminosäuren gibt, die jeweils unabhängig voneinander durch Y repräsentiert werden, und 63 Gly-X-Y-Sequenzen die gleichen oder unterschiedlich sein können.

9. Rekombinante Gelatine zur Verwendung gemäß irgendeinem der Ansprüche 1 bis 8, wobei die rekombinante Gelatine die in SEQ ID NO: 1 gezeigte Aminosäuresequenz hat.

10. Rekombinante Gelatine zur Verwendung gemäß irgendeinem der Ansprüche 1 bis 9, wobei die rekombinante Gelatine vernetzt ist.

11. Rekombinante Gelatine zur Verwendung gemäß Anspruch 10, wobei die Vernetzung unter Verwendung eines Aldehyds, eines Kondensationsmittels oder eines Enzyms durchgeführt wird.

12. Rekombinante Gelatine zur Verwendung gemäß irgendeinem der Ansprüche 1 bis 11, welche sich an einer Angiogenesestelle ansammelt, um Angiogenese zu induzieren.

13. Verwendung einer rekombinanten Gelatine mit einer Aminosäuresequenz, abgeleitet von einer partiellen Aminosäuresequenz von Kollagen, zur Produktion eines Angiogenese-induzierenden Medikaments zur Behandlung von ischämischen Erkrankungen, diabetischem Gangrän, Geschwür, Hörverlust, Herzerkrankungen, Arteriosklerose, akuten Koronarsyndromen, akutem Myokardinfarkt, instabiler Angina pectoris und plötzlichem Herztod, wobei die rekombinante Gelatine Wiederholungen einer durch Gly-X-Y repräsentierten, für Kollagen charakteristischen Sequenz umfasst und ein einzelnes Molekül der rekombinanten Gelatine wenigstens zwei Zelladhäsionssignalsequenzen aufweist, wobei X und Y jeweils unabhängig voneinander irgendeine Aminosäure repräsentieren und eine Mehrzahl von Gly-X-Y-Sequenzen die gleichen oder unterschiedlich sein können, und wobei die Zelladhäsionssignalsequenz eine Aminosäuresequenz, repräsentiert durch Glu-Arg-Gly-Asp, ist.

## Revendications

1. Gélatine recombinante présentant une séquence d'acides aminés dérivée d'une séquence d'acides aminés partielle du collagène pour une utilisation dans un procédé de traitement de maladies ischémiques, d'une gangrène diabétique, d'un ulcère, d'une perte d'audition, de maladies cardiaques, d'une artériosclérose, de syndromes coronariens aigus, d'un infarctus aigu du myocarde, d'une angine de poitrine instable, et d'une mort cardiaque subite par induction d'angiogenèse, dans laquelle la gélatine recombinante comprend des répétitions d'une séquence représentée par Gly-X-Y caractéristique du collagène et une molécule simple de la gélatine recombinante présente au moins deux séquences de signal d'adhésion cellulaire, dans laquelle X et Y représentent chacun indépendamment un quelconque acide aminé et une pluralité de séquences Gly-X-Y peuvent être identiques ou différentes, et dans laquelle la séquence de signal d'adhésion cellulaire est une séquence d'acides aminés représentée par Glu-Arg-Gly-Asp.

2. Gélatine recombinante pour une utilisation selon la revendication 1, dans laquelle la gélatine recombinante comprend des répétitions d'une séquence représentée par Gly-X-Y caractéristique du collagène et présente une masse moléculaire de 2 kDa à 100 kDa, dans laquelle X et Y représentent chacun indépendamment un quelconque acide aminé, et une pluralité de séquences Gly-X-Y peuvent être identiques ou différentes.

3. Gélatine recombinante pour une utilisation selon la revendication 1 ou 2, dans laquelle la gélatine recombinante comprend des répétitions d'une séquence représentée par Gly-X-Y caractéristique du collagène et présente une masse moléculaire de 10 kDa à 90 kDa, dans laquelle X et Y représentent chacun indépendamment un quelconque acide aminé, et une pluralité de séquences Gly-X-Y peuvent être identiques ou différentes.

4. Gélatine recombinante pour une utilisation selon l'une quelconque des revendications 1 à 3, dans laquelle la séquence d'acides aminés de la gélatine recombinante ne comprend aucune de la sérine et de la thréonine.

5. Gélatine recombinante pour une utilisation selon l'une quelconque des revendications 1 à 4, dans laquelle la séquence d'acides aminés de la gélatine recombinante ne comprend aucune de la sérine, la thréonine, l'asparagine, la tyrosine, et la cystéine.

6. Gélatine recombinante pour une utilisation selon l'une quelconque des revendications 1 à 5, dans laquelle la séquence d'acides aminés de la gélatine recombinante ne comprend pas une séquence d'acides aminés représentée par Asp-Arg-Gly-Asp.

7. Gélatine recombinante pour une utilisation selon l'une quelconque des revendications 1 à 6, dans laquelle la gélatine recombinante est représentée par la formule suivante :
A-[(Gly-X-Y)ₙ]ₘ-B
dans laquelle A représente un quelconque acide aminé ou une séquence d'acides aminés, B représente un quelconque acide aminé ou une quelconque séquence d'acides aminés, il existe n acides aminés chacun indépendamment représenté par X, il existe n acides aminés représentés chacun indépendamment par Y, n représente un entier de 3 à 100, m représente un entier de 2 à 10, et n séquences Gly-X-Y peuvent être identiques ou différentes.

8. Gélatine recombinante pour une utilisation selon l'une quelconque des revendications 1 à 7, dans laquelle la gélatine recombinante est représentée par la formule suivante :
Gly-Ala-Pro-[(Gly-X-Y)₆₃]₃-Gly
dans laquelle il existe 63 acides aminés représentés chacun indépendamment par X, il existe 63 acides aminés représentés chacun indépendamment par Y, et 63 séquences Gly-X-Y peuvent être identiques ou différentes.

9. Gélatine recombinante pour une utilisation selon l'une quelconque des revendications 1 à 8, dans laquelle la gélatine recombinante présente la séquence d'acides aminés montrée dans ID SEQ NO : 1.

10. Gélatine recombinante pour une utilisation selon l'une quelconque des revendications 1 à 9, dans laquelle la gélatine recombinante est réticulée.

11. Gélatine recombinante pour une utilisation selon la revendication 10, dans laquelle une réticulation est réalisée en utilisant un aldéhyde, un agent de condensation, ou une enzyme.

12. Gélatine recombinante pour une utilisation selon l'une quelconque des revendications 1 à 11, qui s'accumule au niveau d'un site d'angiogenèse afin d'induire une angiogenèse.

13. Utilisation d'une gélatine recombinante présentant une séquence d'acides aminés dérivée d'une séquence d'acides aminés partielle du collagène pour une production d'un médicament inducteur d'angiogenèse pour le traitement de maladies ischémiques, d'une gangrène diabétique, d'un ulcère, d'une perte d'audition, de maladies cardiaques, d'une artériosclérose, de syndromes coronariens aigus, d'un infarctus aigu du myocarde, d'une angine de poitrine instable, et d'une mort cardiaque subite, dans laquelle la gélatine recombinante comprend des répétitions d'une séquence représentée par Gly-X-Y caractéristique du collagène et une molécule simple de la gélatine recombinante présente au moins deux séquences de signal d'adhésion cellulaire, dans laquelle X et Y représentent chacun indépendamment un quelconque acide aminé et une pluralité de séquences Gly-X-Y peuvent être identiques ou différentes, et dans laquelle la séquence de signal d'adhésion cellulaire est une séquence d'acides aminés représentée par Glu-Arg-Gly-Asp.


### SEQUENCE LISTING

<110> FUJIFILM Corporation
<120> An agent for induction of angiogenesis comprising a recombinant
   gelatin
<130> 101057A
<160> 1
<170> PatentIn version 3.5
<210> 1
   <211> 571
   <212> PRT
   <213> Recombinant
<400> 1
   Gly Ala Pro Gly Ala Pro Gly Leu Gln Gly Ala Pro Gly Leu Gln Gly
   1               5                   10                  15
   Met Pro Gly Glu Arg Gly Ala Ala Gly Leu Pro Gly Pro Lys Gly Glu
               20                  25                  30
   Arg Gly Asp Ala Gly Pro Lys Gly Ala Asp Gly Ala Pro Gly Ala Pro
           35                  40                  45
   Gly Leu Gln Gly Met Pro Gly Glu Arg Gly Ala Ala Gly Leu Pro Gly
       50                  55                  60
   Pro Lys Gly Glu Arg Gly Asp Ala Gly Pro Lys Gly Ala Asp Gly Ala
   65                  70                  75                  80
   Pro Gly Lys Asp Gly Val Arg Gly Leu Ala Gly Pro Ile Gly Pro Pro
                   85                  90                  95
   Gly Glu Arg Gly Ala Ala Gly Leu Pro Gly Pro Lys Gly Glu Arg Gly
               100                 105                 110
   Asp Ala Gly Pro Lys Gly Ala Asp Gly Ala Pro Gly Lys Asp Gly Val
           115                 120                 125
   Arg Gly Leu Ala Gly Pro Ile Gly Pro Pro Gly Pro Ala Gly Ala Pro
       130                 135                 140
   Gly Ala Pro Gly Leu Gln Gly Met Pro Gly Glu Arg Gly Ala Ala Gly
   145                 150                 155                 160
   Leu Pro Gly Pro Lys Gly Glu Arg Gly Asp Ala Gly Pro Lys Gly Ala
                   165                 170                 175
   Asp Gly Ala Pro Gly Lys Asp Gly Val Arg Gly Leu Ala Gly Pro Pro
               180                 185                 190
   Gly Ala Pro Gly Leu Gln Gly Ala Pro Gly Leu Gln Gly Met Pro Gly
           195                 200                 205
   Glu Arg Gly Ala Ala Gly Leu Pro Gly Pro Lys Gly Glu Arg Gly Asp
       210                 215                 220
   Ala Gly Pro Lys Gly Ala Asp Gly Ala Pro Gly Ala Pro Gly Leu Gln
   225                 230                 235                 240
   Gly Met Pro Gly Glu Arg Gly Ala Ala Gly Leu Pro Gly Pro Lys Gly
                   245                 250                 255
   Glu Arg Gly Asp Ala Gly Pro Lys Gly Ala Asp Gly Ala Pro Gly Lys
               260                 265                 270
   Asp Gly Val Arg Gly Leu Ala Gly Pro Ile Gly Pro Pro Gly Glu Arg
           275                 280                 285
   Gly Ala Ala Gly Leu Pro Gly Pro Lys Gly Glu Arg Gly Asp Ala Gly
       290                 295                 300
   Pro Lys Gly Ala Asp Gly Ala Pro Gly Lys Asp Gly Val Arg Gly Leu
   305                 310                 315                 320
   Ala Gly Pro Ile Gly Pro Pro Gly Pro Ala Gly Ala Pro Gly Ala Pro
                   325                 330                 335
   Gly Leu Gln Gly Met Pro Gly Glu Arg Gly Ala Ala Gly Leu Pro Gly
               340                 345                 350
   Pro Lys Gly Glu Arg Gly Asp Ala Gly Pro Lys Gly Ala Asp Gly Ala
           355                 360                 365
   Pro Gly Lys Asp Gly Val Arg Gly Leu Ala Gly Pro Pro Gly Ala Pro
       370                 375                 380
   Gly Leu Gln Gly Ala Pro Gly Leu Gln Gly Met Pro Gly Glu Arg Gly
   385                 390                 395                 400
   Ala Ala Gly Leu Pro Gly Pro Lys Gly Glu Arg Gly Asp Ala Gly Pro
                   405                 410                 415
   Lys Gly Ala Asp Gly Ala Pro Gly Ala Pro Gly Leu Gln Gly Met Pro
                   420                         425                         430
   Gly Glu Arg Gly Ala Ala Gly Leu Pro Gly Pro Lys Gly Glu Arg Gly
               435                         440                         445
   Asp Ala Gly Pro Lys Gly Ala Asp Gly Ala Pro Gly Lys Asp Gly Val
           450                         455                         460
   Arg Gly Leu Ala Gly Pro Ile Gly Pro Pro Gly Glu Arg Gly Ala Ala
   465                 470                         475                         480
   Gly Leu Pro Gly Pro Lys Gly Glu Arg Gly Asp Ala Gly Pro Lys Gly
                   485                         490                         495
   Ala Asp Gly Ala Pro Gly Lys Asp Gly Val Arg Gly Leu Ala Gly Pro
                   500                         505                         510
   Ile Gly Pro Pro Gly Pro Ala Gly Ala Pro Gly Ala Pro Gly Leu Gln
               515                         520                         525
   Gly Met Pro Gly Glu Arg Gly Ala Ala Gly Leu Pro Gly Pro Lys Gly
           530                         535                         540
   Glu Arg Gly Asp Ala Gly Pro Lys Gly Ala Asp Gly Ala Pro Gly Lys
   545                 550                         555                         560
   Asp Gly Val Arg Gly Leu Ala Gly Pro Pro Gly
                   565                         570